# EUROPEAN PATENT APPLICATION

(11) **EP 4 759 265 A1**
(43) Date of publication of application: **17.06.2026**
(21) Application number: 24850425.0
(22) Date of filing: 08.08.2024
(51) Int. Cl.: A61F 2/00, A61F 2/02, A61F 2/04, A61F 2/06, A61F 2/82, B29C 41/00, B29C 41/02, B29C 41/14

(54) **SYSTEM FOR PRODUCING VASCULAR PROSTHESES**

(30) Priority: 09.08.2023 CL 202302356
(71) Applicant: Cells For Cells S.A., Santiago 7550101 (CL)
(72) Inventor: BELGERI ROJAS, Vicente, Santiago, 7550101 (CL); NOVOA ALVEAR, Javier, Santiago, 7550101 (CL); ACEVEDO COX, Juan Pablo, Santiago, 7550101 (CL); BASAURE, Sebastián, Santiago, 7550101 (CL)
(74) Representative: Elzaburu S.L.P.
(86) International application number: PCT/CL2024/050087
(87) International publication number: WO 2025/030250

(57) **Abstract**

The present invention consists of an installation for the manufacture of vascular prostheses or grafts comprising an enclosed area; an extractor hood; a support structure arranged inside the enclosure; a mandrel assembly, a core stem of a vascular prosthesis held at one end by said mandrel assembly and a stem positioning assembly comprising the electromechanical and robotic means of displacement to move, support and orient, as an effector, said mandrel assembly and said stem in a first, second, third and fourth immersion position; a nanofiber ejector assembly and a nanofiber ejector positioner assembly comprising the electromechanical and robotic displacement means to displace, support and orient, as an effector, said nanofiber ejector assembly; a first, second and third immersion container arranged in said first, second and third immersion position in said support structure; an immersion container with heater and UV light curing media arranged in said fourth position on said support structure; and a control console.

## Description

### TECHNICAL FIELD

The present invention relates to the production of vascular prostheses, in particular, to a facility for the manufacture of vascular prostheses in a controlled manner and suitable for biomedical applications.

### BACKGROUND TO THE INVENTION

The manufacture of vascular prostheses has been an area of research and development in the medical field. These prostheses, also known as vascular grafts, play a crucial role in the treatment of various vascular diseases, including arterial occlusion, aneurysms, and arteriovenous malformations. They are also used in patients with chronic or end-stage renal disease requiring hemodialysis treatments, to produce a vascular access with an arteriovenous graft using a vascular prosthesis as a bridge, replacing an arteriovenous fistula.

Several procedures have been developed for biomedical applications using prostheses or vascular grafts. These grafts are collected from the patient himself and have certain advantages such as biocompatibility and low incidence of infections, however, also with limitations, such as adequate vasculature and the invasiveness of the procedure. Subsequently, synthetic vascular prostheses were introduced, made of materials such as polyester (Dacron) and expanded polytetrafluoroethylene (ePTFE). These materials showed excellent biocompatibility and wear resistance, which allowed the manufacture of grafts with specific characteristics for each patient and each application. However, these synthetic materials also have their own limitations, such as the tendency to thrombosis and stenosis.

Current vascular prostheses, whether synthetic or biological, have limitations in terms of availability, cost, variability, and postoperative complications. Synthetic prostheses, such as those made of ePTFE or polyester, often have problems related to thrombus formation, intimal hyperplasia, atherosclerosis, and infections. Biological prostheses, although superior, also have limitations, such as their partial availability, high cost and high degree of variability.

Below, some relevant publications of the state of the art related to the production of vascular prostheses for biomedical applications are discussed.

### Previous art

The U.S. patent application publication US20120232643A1 describes tubular casting processes, such as dip coating, that can be used to form substrates from polymer solutions to manufacture implantable devices, such as stents. Operating parameters are established, such as the number of immersions, the duration of the time of each immersion within a solution, as well as the delay time between each immersion or the drying or curing time between immersions and chuck removal speeds from the solution that can each be controlled to vary the mechanical characteristics. Thus, this publication refers in particular to the operating parameters in a process in a manufacturing of dip-coating additive to ensure its mechanical strength and ductility to prevent or inhibit brittle fracture, when used as a stent, for implantation inside a patient's body. However, the technical means of producing the implants are briefly mentioned as a base that can support a spine that houses a drive column and a support arm. A motor can drive the drive column vertically along the column to move the support arm accordingly. A mandrel is attached to the support arm above the vessel that can be filled with a polymeric solution (e.g., PLLA, PLA, PLGA, etc.) into which the mandrel can be immersed using a linear motion. Additional motors are mentioned to impart another rotation to the mandrel, and the possibility of providing a controlled atmosphere, with a limit of relative humidity and immersion temperature. Thus, it is observed that the means of production developed in this document are mainly restricted to the process of coating by immersion with the operating parameters described therein.

The inventors of the present invention have developed the invention described in the U.S. patent application publication, US10688694, which describes a versatile method for manufacturing multilayer hollow tubes using a layer-by-layer stem immersion approach and using different biomaterials. Precise control of manufacturing parameters such as up/down speeds, bar rotation speed, and cross-linking or polymerization time is allowed. This technology allows for the generation of more complex multilayer hollow tubes, such as systemic vasculature-like structures, urethral grafts, prostate grafts, and the like.

These same inventors have also published their previous work in the following publications:
- "Akentjew, T. L., Terraza, C., Suazo, C., Maksimcuka, J., Wilkens, C. A., Vargas, F., ... & Valenzuela, L. M. (2019). Rapid fabrication of reinforced and cell-laden vascular grafts structurally inspired by human coronary arteries. Nature communications, 10(1), 1-15. https://doi.org/10.1038/s41467-019-11090-3 "
- "Wilkens, C. A., Rivet, C. J., Akentjew, T. L., Alverio, J., Khoury, M., & Acevedo, J. P. (2016). Layer-by-layer approach for a uniformed fabrication of a cell patterned vessel-like construct. Biofabrication, 9(1), 015001. https://doi.org/10.1088/1758-5090/9/1/015001 "

Thus, the inventors of the present invention have previously developed a method and a vascular prosthesis with a multilayer structure, an appropriate fiber orientation. The parameters disclosed in these publications allow the establishing of the necessary biomaterials, the appropriate concentrations, temperature ranges, flow of solutions and some other variables of manufacturing processes. However, the robotic device for the production of these vascular prostheses is only described in a summary way and illustrated in an extremely schematic way. Robotic device designs are presented completely restricted in their possibilities and to the steps of the production method widely discussed in these publications. This presents the disadvantages of a rigid design suitable only for that specific method and an oversimplified conception which will have to be modified when trying to reproduce itself. In fact, the description of its general components in terms of the use of various platforms, pulleys, belts, stepper motors controlled by electronic circuits, does not allow us to visualize an advantageous design that allows for satisfying the requirements in vascular prostheses for their respective biomedical applications.

In addition, in order to design a reliable manufacturing apparatus to produce viable vascular prostheses for biomedical applications, it must allow the production of prostheses that meet certain acceptance criteria, for example, the following criteria can be considered:
▪ The vascular prosthesis must have a wall thickness of 0.1mm ±10%.
▪ An endotoxin assessment of a cross-section of the stent should be within an endotoxin limit of 0.5 EU/mL or 20 EU/prosthesis.
▪ The vascular prosthesis should not have a dissection of its multilayers.
▪ The vascular prosthesis must have an adequate wall structure, without imperfections such as cracks or bubbles.
▪ The vascular prosthesis must have an adequate overall structure evaluated by a macroscopic examination.

If these criteria are not met, the produced vascular prosthesis and raw materials must be discarded, a cleaning process and calibration of the production apparatus must be carried out.

### Technical problem

Although there has been a certain level of automation in the means of manufacture proposed as robotic devices to manufacture vascular prostheses, there are still several drawbacks that must be overcome to achieve a scale-up and industrial systematization in the production of these devices, in addition to the strict quality controls which must be overcome to adapt to the biomedical applications for which they are intended.

The robotic devices for making vascular prostheses from previous art feature a stem in an upright position that is immersed in a container of slender proportions, also vertically oriented. The solutions contained in this container require constant temperature and some type of agitation to maintain these homogeneous solutions in the concentration of their components throughout the volume of the container to ensure an even immersion coating throughout the stem. The designs of prosthetic fabrication apparatus proposed and simplified from the previous art do not indicate all the means of control, temperature control, or agitation that should be used in these devices. Thus, the intervention of operators would be necessary in many stages of the methods developed to manufacture a vascular prosthesis, which can result in a source of defect due to variability between prostheses or contamination of the final product.

On the other hand, possible spills and drips of the solutions from the container tubes or from the stem, in the process of immersion of the stem or in the subsequent stage of coating by filament blowing, are not considered, so a continuous operation, at an industrial level, of the robotic devices to manufacture vascular prostheses of the previous art would generate great losses of material, making the product more expensive and reducing the performance of the process.

Therefore, there is a clear need to develop an automated device or machine that allows for the efficient production of reliable and affordable vascular prostheses, which meet the stated acceptance criteria and all those that are necessary for the biomedical application for which they are intended, for example, for patients receiving hemodialysis to treat end-stage renal disease and for other biomedical applications. Scalability and reproducibility remain significant challenges in biomedical applications where each implant can be unique.

### Technical solution

To address the problems mentioned above, an object of the present invention is an installation for the manufacture of vascular prostheses or grafts that operates autonomously, repetitively and controlled, in order to achieve scalability and reduce the costs associated with developing a vascular prosthesis, while guaranteeing the quality of the prostheses manufactured. To this end, human intervention is minimized by automating the stages of the methods of production of prostheses, guaranteeing compliance with the necessary acceptance criteria in medical applications. For these purposes, the installation for the manufacture of vascular prostheses includes an enclosure with a controlled atmosphere inside; an extractor hood that allows for controlling a laminar flow of clean air inside the enclosure; a support structure arranged inside the enclosure; a mandrel assembly, a core stem of a vascular prosthesis held at one end by said mandrel assembly and a stem positioner assembly comprising the electromechanical and robotic displacement means to move, support and orient, as an effector, said mandrel assembly and said stem in a first, second, third, fourth and fifth position; a nanofiber ejector assembly and a nanofiber ejector positioner assembly comprising the electromechanical and robotic displacement means to displace, support and orient, as an effector, said nanofiber ejector assembly; a first, second and third immersion container arranged in said first, second and third immersion position in said support structure; an immersion container with heater and UV light curing media arranged in said fourth position on said support structure; and a control console.

### Advantages of the present invention

The present invention makes it possible to reduce costs and increase the availability of vascular prostheses through the automation of artisanal and laboratory processes to date by means of electromechanics, robotics and controlled environments. This made it difficult to implement it in biomedical applications. Thus, the present invention makes it possible to manufacture vascular prostheses without the intervention of an operator during the combined manufacturing stages by means of immersion coatings and by projection of nanofibers by blowing compressed air, ensuring the final quality of the prosthesis.

The present invention makes it possible to manufacture a vascular prosthesis in less than an hour, even in up to 30 minutes complying with the aforementioned acceptance criteria and an assembly with bioinspired structural configurations of concentric cell patterns with fibers at specific angles and wavy arrangements.

The present invention permits the fabrication of small-caliber vascular grafts or prostheses that exhibit the "J"-shaped mechanical response and flexibility of human coronary arteries.

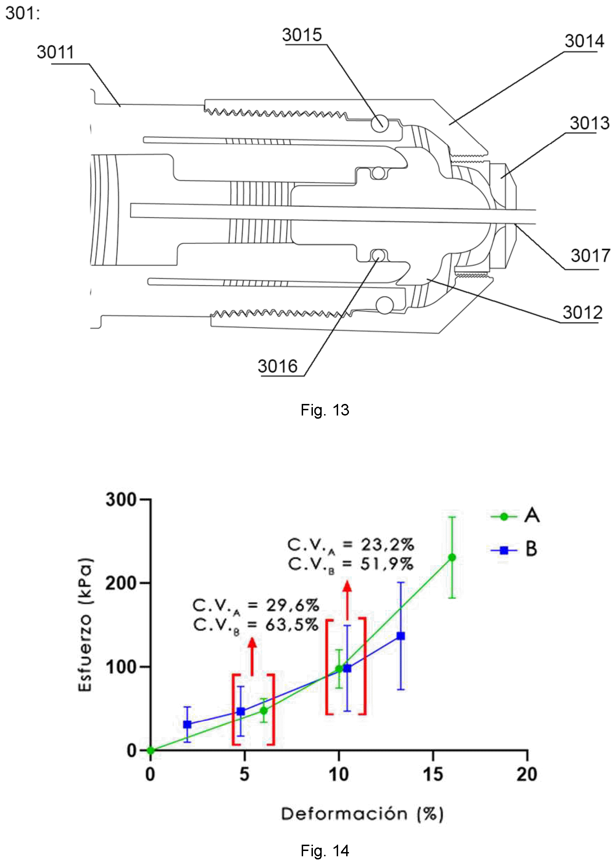

Comparative studies carried out by the inventors show the advantages that the present invention offers in comparison with the apparatus for the manufacture of vascular prostheses disclosed in the patent of invention of these same inventors, US10688694. These studies show an improvement in that the present invention allows a lower variability of the mechanical resistance of the grafts produced, allowing better reliability and precision in manufacturing. These experimental results presented in Figure 14 are discussed in the detailed description of the invention.

### BRIEF DESCRIPTION OF THE FIGURES

The figures of preferred forms of realization of the present invention are described below. It should be noted that the figures are provided only as illustrations and that the invention is not limited by these illustrations. The figures are not necessarily to scale and some features may be exaggerated or minimized to show details of particular components. Well-known components, materials, or methods are not necessarily described in great detail to avoid obscuring the present description. Any specific structural and functional detail described here should not be interpreted as limiting, but simply as a basis for claims and as a representative basis for teaching a skilled artisan how to use the invention in a diverse way.
Figure 1 schematically represents, in a frontal view, an installation for the manufacture of vascular prostheses according to a form of realization of the present invention.
Figure 2 schematically represents, in a side view, the installation for the manufacture of vascular prostheses in Figure 1.
Figure 3 schematically represents the installation for the manufacture of vascular prostheses in Figure 1 in a different operating position.
Figure 4 schematically represents the installation for the manufacture of vascular prostheses in Figure 1 in a different operating position.
Figure 5 schematically represents the installation for the manufacture of vascular prostheses in Figure 1 in yet another different operating position.
Figure 6 schematically represents, in a frontal view, an installation for the manufacture of vascular prostheses according to another form of realization of the present invention.
Figure 7 schematically represents, in a frontal view, an installation for the manufacture of vascular prostheses according to an additional form of realization of the present invention.
Figure 8 schematically represents, in a top view, the installation for the manufacture of vascular prostheses in Figure 7.
Figure 9 schematically depicts, in a front view, an installation for the manufacture of vascular prostheses according to yet another additional form of realization of the present invention.
Figure 10 schematically represents, in an isometric view, a container of solution for immersion present in the installation for the manufacture of vascular prostheses in figures 7 and 8.
Figure 11 illustrates, in an exploded view, a blown nanofiber ejector assembly to the present invention.
Figure 12 illustrates, in a perspective view, the blown nanofiber ejector assembly in Figure 11.
Figure 13 illustrates, in a cross-sectional view, the nozzle of the blown nanofiber ejector assembly in Figures 11 and 12.
Figure 14 illustrates a comparative graph of stress versus deformation of grafts produced with the present invention and according to previous art.

### DETAILED DESCRIPTION OF THE INVENTION

In the following detailed description of the invention, reference is made to the attached figures that are part of this detailed description. The illustrative forms of realization described in this detailed description and represented in the figures are not intended to be limiting. Other forms of embodiment may be used and other changes may be made without departing from the spirit or scope of the present invention as defined in the attached claims.

In this document, vascular prostheses or grafts are defined as devices and materials that are used to replace or repair vascular tissue, including, for example, tubular constructs, patches, and other devices that are intended to create barriers that allow blood to circulate in contact with blood.

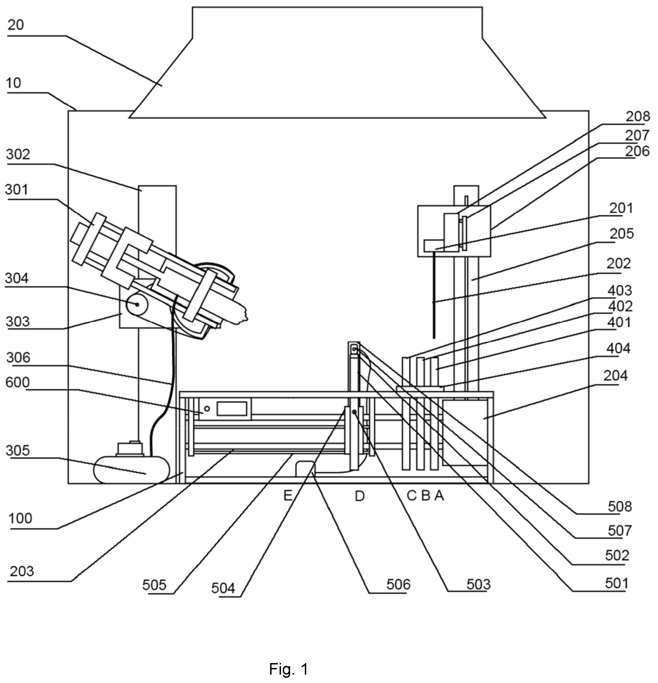

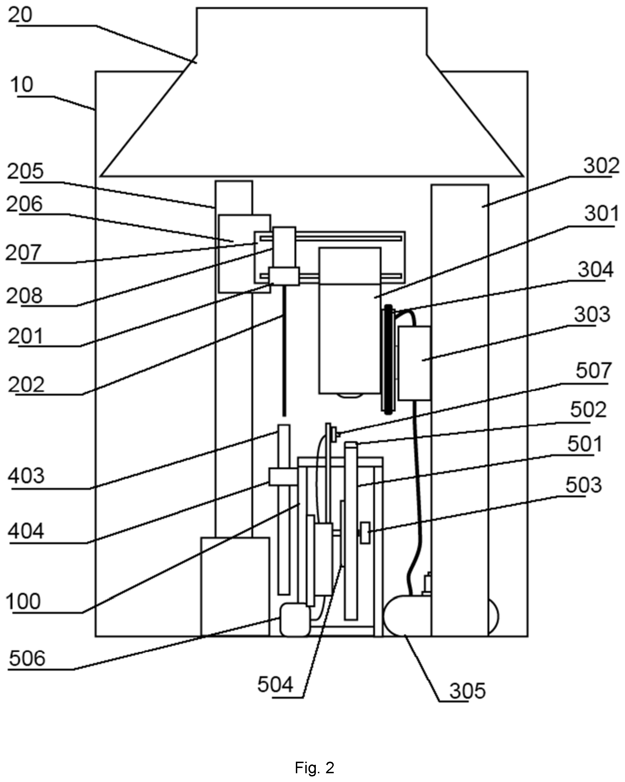

With reference to Figures 1 and 2, the present invention consists of an installation for the manufacture of prosthetic or vascular grafts comprising:
an enclosure (10) closed with air inlets with filters that allows the atmosphere and its microorganism content to be controlled and to provide a sterile and closed environment inside;
an extractor hood (20) with a filter for particles and/or microorganisms, biosafety, hermetic and with good manufacturing practice (GMP) operation that allows a clean air flow to be controlled inside the enclosure (10);
a support structure (100) arranged inside the enclosure (10);
a mandrel or rotary support assembly (201), a vascular prosthesis core stem (202) held at one end by said mandrel assembly (201) and a stem positioning assembly comprising the electromechanical and robotic displacement means (203, 204, 205, 206, 207, 208) to move, support and orient, as an effector, said mandrel assembly (201) and said stem (202) in a first, second, third, fourth and fifth position (A, B, C, D and E);
a nanofiber ejector assembly (301) and a nanofiber ejector positioning assembly comprising the electromechanical and robotic displacement means (302, 303, 304) for moving, supporting and orienting, as an effector, said nanofiber ejector assembly by blowing (301);
a first, second and third immersion container (401, 402, 403) arranged in said first, second and third position (A, B, C) in said support structure (100);
an immersion container (501) with heater and UV light curing media (506, 507, 508) arranged in said fourth position (D) on said support structure (100); and
a control console (600) comprising computational media, memory, access media such as keyboards and controllers, storage drive readers, and communication media for wired or wireless connection to the controllers of the electromechanical and robotic displacement media (203, 204, 205, 206, 207, 208, 302, 303, 304), the immersion container heater (501), and the UV curing media.

In an exemplary way, this immersion container (501) with heater is configured to keep the contained solution at a temperature of around 37°C.

Preferably, said enclosed enclosure (10) has an airtight environment that is not in contact with the outside, and is arranged in a room that allows compliance with the standards of good manufacturing practices (GMP) such as controlled access and special cleaning, with sterilization means, such as UV light and a separate compartment of the enclosure, to sterilize solid materials before entering the enclosure such as supplies and accessories.

Preferably, this extractor hood (20) produces a laminar flow from the enclosure (10).

Preferably, said electromechanical and robotic means of displacement of said stem positioner assembly comprise a horizontal movable stem positioner support (204); a stem positioner lifting pillar (205); a movable stem positioner lift stand (206); shank positioner cross rails (207); a transverse movable stem positioner bracket (208) and stem plunger bottom rails (203).

Preferably, such electromechanical and robotic means of displacement of said nanofiber ejector positioner assembly comprise an ejector positioner lifting pillar (302); an ejector positioner angular variation unit (304); A mobile ejector positioner lift stand (303).

According to a preferred embodiment of the present invention, the installation for the manufacture of vascular prostheses comprises an air compressor (305) located outside the enclosure (10) and connected to the nanofiber ejector assembly (301) by a compressed air line or hose (306). Alternatively, a compressed air line available in the enclosure (10) can feed compressed air to that nanofiber ejector assembly (301) instead of that hose (306). In an exemplary way, the compressed air is delivered at a pressure of around 4 bar (80 PSI). In particular, these first, second and third immersion containers (401, 402, 403) are arranged on a support fixed to the support structure (100) and, optionally, with an immersion tube collector (404) to recover spills of polymeric solution and facilitate the maintenance of the apparatus.

Alternatively, such first, second and third immersion containers (401, 402, 403) comprise temperature control and agitation means.

In particular, said immersion container (501) in said support structure (100), is supported by a container positioner assembly with electromechanical and robotic displacement means (504, 505) to locate said immersion tube in a position of use (F) and in a position of withdrawal (G), said positioning assembly includes lower and longitudinal rails (505); A mobile tube positioner assembly stand (504).

According to one embodiment, these UV light curing media comprise a UV LED panel mounted directly on the structure (100) to illuminate said core stem of the vascular prosthesis (202) when it is placed in said fourth position (D) and immersed in said immersion container (501). Alternatively, such UV light curing media comprise a UV light power supply (506); a guide with UV light (507) powered by said power supply (506) and supported on a UV light holder (508) in said structure (100). For example, a commercial device known as the OmniCure^{®} S2000 can be used as UV light curing media, with a UV light holder (508) arranged so that the UV light guide (507) is located around 2 cm from the stem (201), with a wavelength of 365 nm and power of 1.21 W.cm⁻².

In particular, and without being illustrated, that mandrel or rotary holder assembly (201) comprises a mandrel housing, a stepper motor connected (wirelessly or by cables) to that control console (600) and a mandrel driven by that motor which rotatively holds said stem (202) which may have a diameter of less than 5 mm, for example, a diameter of around 4 mm, even a diameter of around 0.5 mm useful in vascular microsurgery, and a variable length, for example, of at least 15 cm. Other sizes of this stem (202) are possible, for example, a diameter of around 10 mm, even around 50 mm, for a vascular patch fabrication.

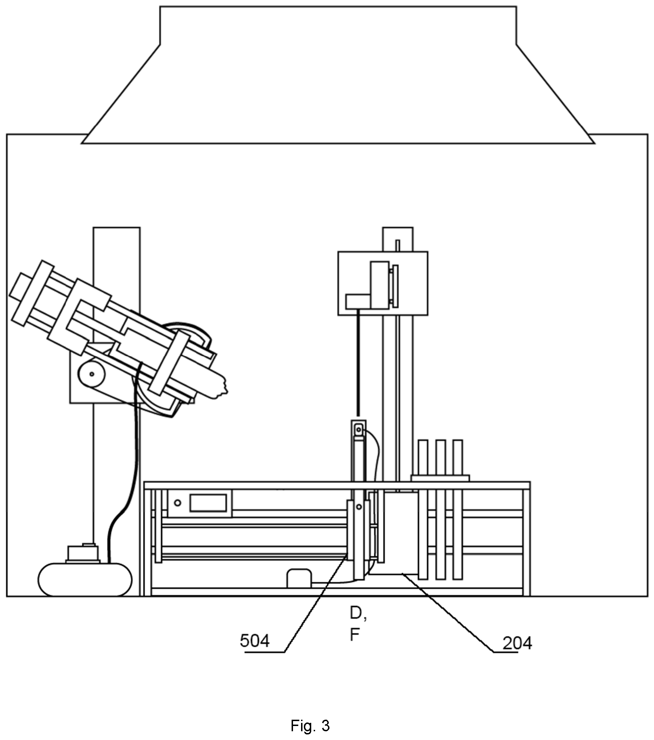

As can be seen in Figures 1, 3, 4 and 5, the stem positioner assembly can place the stem (202) in the respective positions (A, B, C, D and E), thanks to the controlled movement of the horizontal movable stem positioner support (204) to carry out immersion stages in different solutions of the respective containers (401, 402, 403 and 501), by moving the stem (202) in a controlled manner according to the stages of the methods described in the previous art, e.g. in Document US10688694. Preferably, said nanofiber ejector positioner assembly (301)

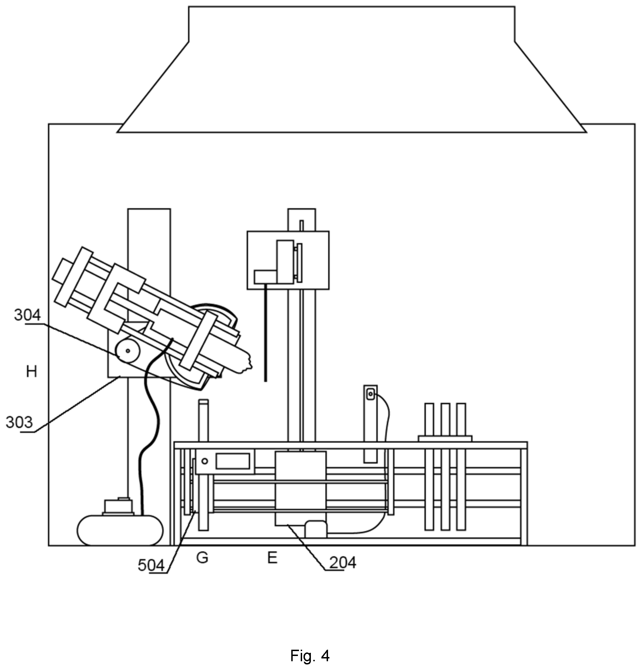

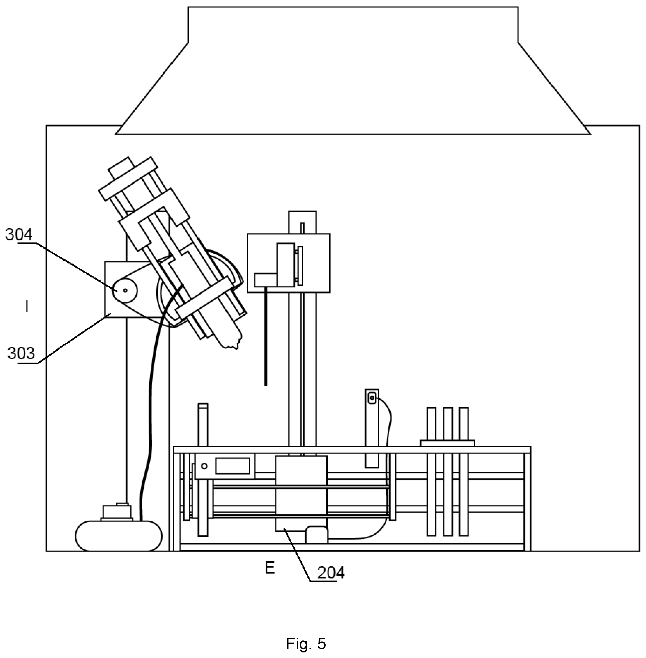

As can be seen in Figures 4 and 5, the stem positioner assembly is also positioned in position (E), so that the nanofiber ejector positioner assembly arranges the nanofiber ejector assembly (301), in the position (H) of the first height with a first angular orientation, thanks to the ejector positioner angular variation unit (304) and the ejector positioner positioner lifting mobile support (303). In this position (H) of first height and angular orientation, the nanofiber ejector assembly (301) can eject a fiber by means of pressure applied to a syringe with a polymer, e.g. Polycaprolactone (PLC) and blowing compressed air circulating annularly to the syringe, e.g. compressed air at 4 bar, at an angle of incidence with respect to the stem (202) of 21°, with its nozzle at a distance L1 from the core stem (201), for example, of around 30 cm. In Figure 5, it can be seen that the stem positioner assembly is kept in position (E), and the mobile ejector positioner lifting support (303) is raised to the second height position (I) with a second angular orientation thanks to this ejector positioner angular variation unit (304), where the angle of incidence of the ejected fiber on the stem (202) increases to 67°, according to the stages of the methods described in the previous art, for example, in document US10688694.

According to a first embodiment method illustrated in Figures 1 to 6, the containers (401, 402, 403, 501) with immersion solution are copper or stainless steel tubes arranged vertically, and the first, second, third and fourth positions (A, B, C, and D) of the stem (202) are aligned vertically and on a first, second, third and fourth respective vertical pipes (401, 402, 403, 501).

According to this first method of construction, this immersion container (501) includes a lid with automatic closure (502) and a rotary actuator (503) that allows the immersion container (501) to be rotated on a transverse axis, for the agitation of the solution in the container (501).

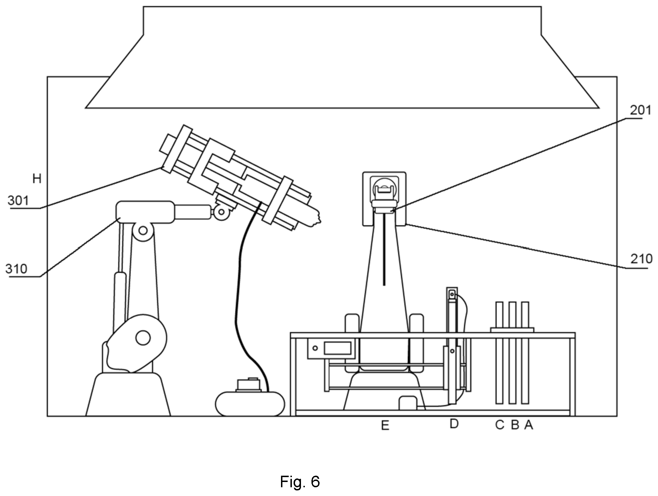

Alternatively, and as illustrated in Figure 6, said stem positioning assembly comprises a robotic manipulator arm (210), e.g., 6-axis as an electromechanical and robotic means of travel, where the mandrel assembly (201) is arranged as the effector of said manipulator arm, and said nanofiber ejector positioner assembly also comprises a robotic manipulator arm (310), for example, 6-axis as electromechanical and robotic displacement means, where the nanofiber ejector assembly (301) is arranged as the effector of said manipulator arm, both being robotic manipulator arms (210, 310) controlled by said control console (600), to carry out the stages of the methods of manufacturing vascular prostheses.

For example, said stem positioning assembly allows the stem (202) to be moved vertically for immersion in containers (401, 402, 403, 501), e.g. at a speed of 2 mm/sec over a distance of 17 cm. The mandrel assembly (201) allows a rotation to be given to the stem (202) at the same time as it is immersed in the solutions, for example, an alternating rotation, with clockwise rotation of 252° followed by a counterclockwise rotation of 126°, or vice versa during an entire immersion stage.

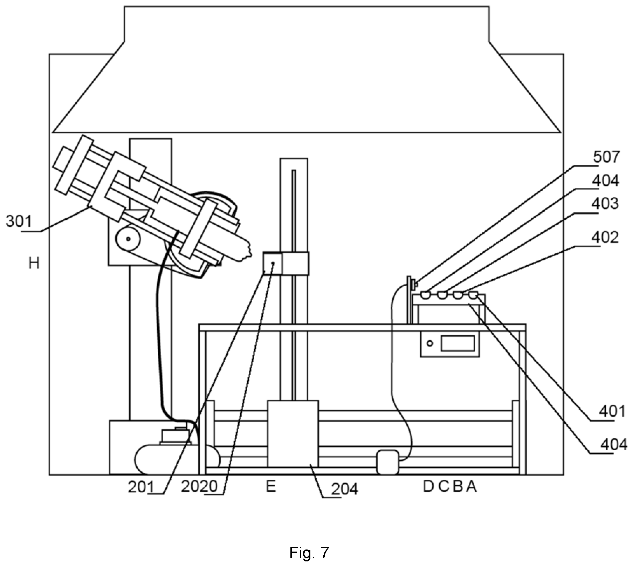

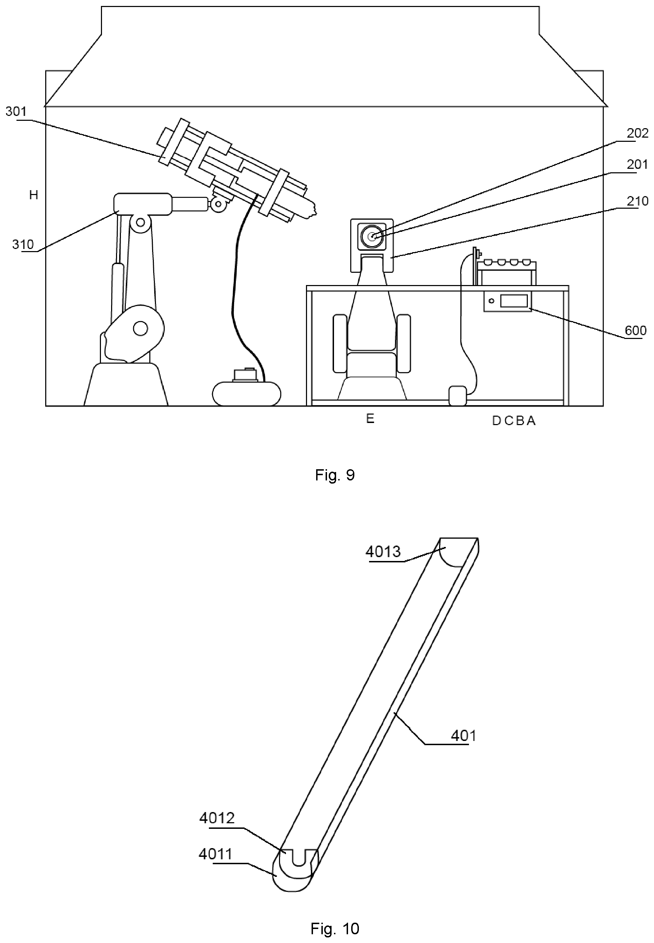

According to a second embodiment method and in reference to figures 7, 8, 9 and 10, the containers (401, 402, 403, 501) with solution for immersion are horizontal channels and the first, second, third and fourth position (A, B, C, and D) of the stem (202) is longitudinally aligned with these channels (401, 402, 403, 501). These channels (better illustrated in Figure 10) may also be made of copper or stainless steel and comprise a cylindrical bottom wall (4011) and a closed distal end (4013) and a proximal end with a passage opening (4012) to the center of the notional cylinder forming the bottom wall. The stem positioning assembly is assembled with said mandrel or rotary support assembly (201) arranged with its axis of rotation and oriented core stem (2020) horizontally, parallel or slightly inclined with respect to the containers (401, 402, 403, 501). The horizontal movable stem positioner bracket (204) is positioned to position the stem (2020) in a fifth position (E) that allows the nanofiber ejector assembly (301) to project a filament onto it. Said core stem may include a notch (2021) that allows the stem to be further lowered through the passage opening (4012) to facilitate the contact of the stem with the contained solution. When the stem is rotated inside one of the containers (401, 402, 403, 501), the solution is dragged by contact with the moment produced in the stem and covers the entire stem, thus achieving the immersion coating stages of vascular prosthesis production methods.

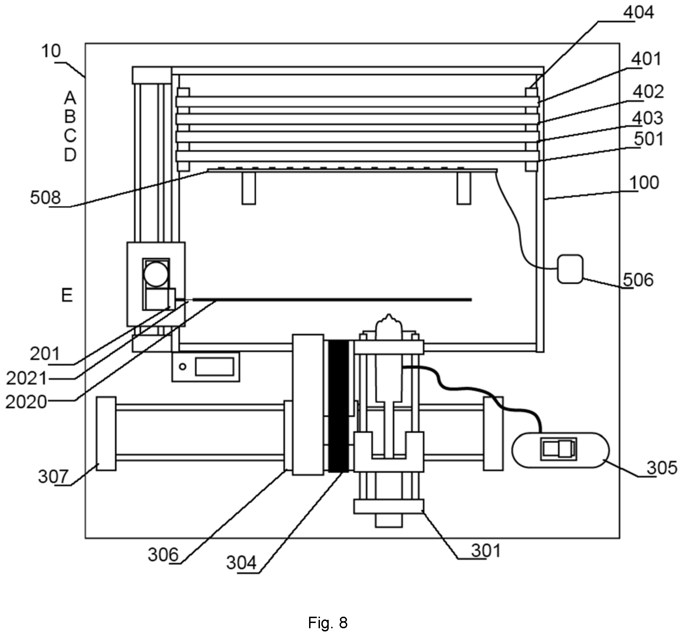

In addition, in this second form of realization, it is contemplated, as illustrated in Figure 8, that the ejector positioner assembly also includes a horizontal mobile support of the ejector positioner (306) on a set of horizontal rails (307) arranged parallel to the stem and the containers (401, 402, 403, 501), so that the nanofiber ejector assembly (301) can travel the length of the stem (2020) in a stage of the production of vascular prostheses, and project at least one nanofiber filament onto said stem, from a position (H) of first height and angular orientation and from a position (I) of second height and angular orientation, which vary in height and angle of incidence of the filament ejected on the stem (2020). For example, this angle of incidence is 21° in a position (H), and 67° in a position (I). Figure 7 illustrates this nanofiber ejector assembly (301) at a position (H) of first height and angular orientation.

Alternatively and similarly to the first embodiment, as illustrated in Figure 9, such a stem positioner assembly comprises a robotic manipulator arm (210), e.g., 6-axis as electromechanical and robotic displacement means, and said nanofiber ejector positioner assembly also comprises a robotic manipulator arm (310), for example, of 6 axes as electromechanical and robotic means of displacement, both arms being robotic manipulators (210, 310) controlled by said control console (600), to carry out the stages of the methods of manufacturing vascular prostheses.

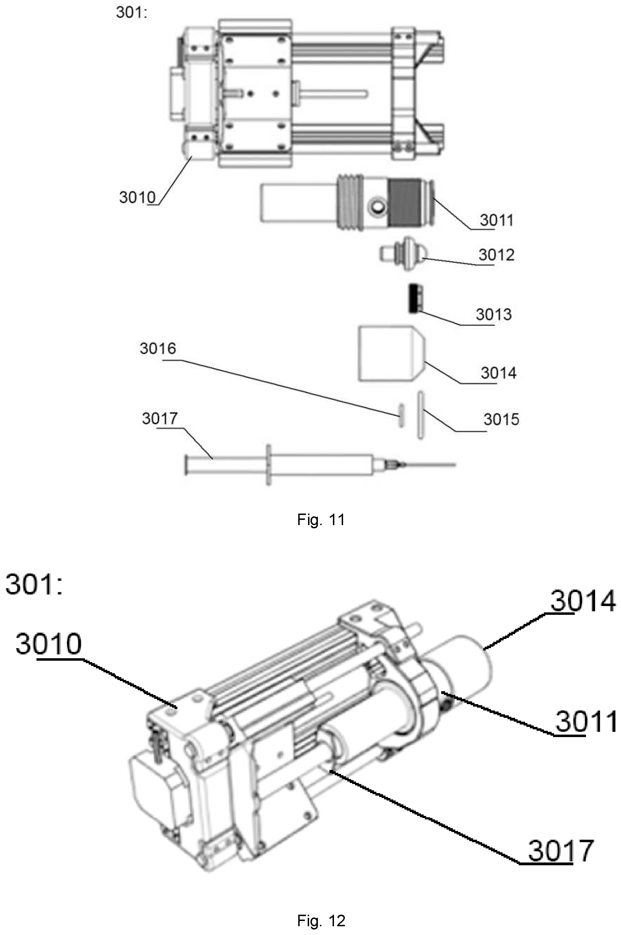

As can be seen in Figures 11, 12 and 13, this nanofiber ejector assembly (301), e.g. Polycaprolactone (PCL) nanofibers, comprises a microfluidic pump consisting of an actuator, e.g. a linear actuator, and a support (3010) housing a main body (3011) with an external compressed air inlet and internal compressed air circulation channels, best seen in Figure 13; a polymer syringe with a metal needle (3017, illustrated in Figure 12 and partially in Figure 13) with Polycaprolactone (PCL) placed in said main body and pressed by said actuator of said microfluidic pump; a cap (3012) of said main body (3011) that prevents the passage of air, that is, it prevents the escape of air through the duct where the syringe is (3017), with a central opening through which the needle of the polymer syringe is arranged (3017), an outer cap (3014) with an internal thread to be coupled with the main body, a nozzle (3013) with an internal thread to mate with the outer cap (3014) and a central opening for the passage of the polymer syringe needle (3017) with a larger diameter annular separation for the passage of compressed air, where the inner face of the nozzle (3013) and the outer face of the cap (3012) form a pointed ring nozzle (aerospike nozzle type) for an air outlet channel; internal seals (3015, 3016) between the plug (3012) and the main body (3011) and between the outer cap (3014) and the main body (3011); where said plug (3012) and said outer cap (3014) form a compressed air circulation channel, of substantially constant cross-sectional area, from the internal compressed air circulation channels to the air outlet channel of said end annular nozzle, said circulation channel follows a mainly curved route.

According to one form of embodiment, this polymer syringe (3017) can be made of plastic. Preferentially, this polymer syringe (3017) is made of glass, which allows for improving the flow and ejection of the polymer through said syringe.

In an exemplary way, this nanofiber blow-off ejector assembly (301) enables a polymer output of around 120 µL/min by advancing the linear actuator of the microfluidic pump and the compressed air flow at around 4 bar.

Preferably, the prosthetic manufacturing facility also includes automatic packaging media that includes automated means of stem removal (202, 2020) and sterile packaging, such as laser cutters for soft ablation at the ends of grafts or vascular prostheses produced on the stem (202, 2020) and a cylindrical stop around its upper end that moves to the lower end pushing the graft out of the stem (202, 2020, 2020). Thus, the manufacture of prostheses or vascular grafts is ensured automatically in the enclosed area (10) without human intervention that could contaminate the product.

### Experimental results:

Figure 14 shows an improvement of the present invention in terms of variability in mechanical strength, plotting the stress versus the deformation of circumferential curves of the vascular grafts produced by the present invention (curve A) and the state-of-the-art apparatus (curve B), described by these inventors in US10688694. Thus, it can be observed that both curves A and B tend to be similar in the stresses that the grafts support in the face of elongation. However, it is also noted that for a strain of 5%, the coefficient of variation of the grafts manufactured with the present invention is C.V._{A} = 29.6%, while the coefficient of variation of grafts manufactured with prostheses manufactured in the state-of-the-art apparatus described in US10688694 is C.V._{B} = 63.5%. It is also noteworthy that for a deformation of 10%, C.V._{A} = 23.2% and C.V._{B} = 51.9%. This indicates that the grafts manufactured in the installation of the present invention have a lower degree of variability compared to the prostheses manufactured in the previous platform. This allows greater precision and reliability in the manufacture of prostheses or vascular grafts.

The terminology used here is intended to describe particular forms of realization only and is not intended to be limiting to the subject revealed. As used here, the singular forms "a", "an", "one" and "the" are intended to include the plural forms as well, unless the context clearly indicates otherwise. The terms "comprises" and/or "which comprises", when used in this specification, shall also be understood to specify the presence of established characteristics, integers, stages, operations, elements and/or components, but do not exclude the presence or addition of one or more characteristics, integers, stages, operations, elements, components and/or groups thereof.

The structures, materials, acts, and corresponding equivalents of all elements of means or stages plus functions in the claims below are intended to include any structure, material, or act to perform the function in combination with other elements claimed as specifically claimed. The description of this disclosed material has been presented for illustrative and descriptive purposes, but is not intended to be exhaustive or limited to the material disclosed in the form disclosed. Many modifications and variations will be evident to experts in the field without departing from the scope and spirit of the revealed topic. The embodiment was chosen and described to better explain the principles of disclosed subject matter and practical application, and to enable other subject matter experts to understand disclosed subject matter for various embodiments with various modifications that are appropriate for the particular use contemplated.

## Claims

1. An installation for the manufacture of prostheses or vascular grafts, **characterized in that** it comprises:
an enclosure (10) with air inlets with filters that allows the atmosphere to be controlled, its content of microorganisms and to provide a sterile and closed environment inside;
an extractor hood with a filter for particles and/or microorganisms (20) that allows a clean air flow to be controlled inside the enclosure (10);
a support structure (100) arranged inside the enclosure (10);
a mandrel or rotary support assembly (201), a vascular prosthesis core stem (202) held at one end by said mandrel assembly (201) and a stem positioning assembly comprising the electromechanical and robotic displacement means (203, 204, 205, 206, 207, 208) to move, support and orient, as an effector, said mandrel assembly (201) and said stem (202) in a first, second, third, fourth and fifth position (A, B, C, D and E);
a nanofiber ejector assembly (301) and a nanofiber ejector positioner assembly comprising the electromechanical and robotic displacement means (302, 303, 304) for moving, supporting and orienting, as an effector, said nanofiber ejector assembly (301);
a first, second and third immersion container (401, 402, 403) arranged in said first, second and third position (A, B, C) in said support structure (100);
an immersion container (501) with heater and UV light curing media (506, 507, 508) arranged in said fourth position (D) on said support structure (100); and
a control console (600) comprising computational media, memory, access media such as keyboards and controllers, storage drive readers, and communication media for wired or wireless connection to the controllers of the electromechanical and robotic displacement media (203, 204, 205, 206, 207, 208, 302, 303, 304), the immersion container heater (501), and the UV curing media.

2. The installation according to claim 1, **characterized in that** said nanofiber ejector assembly (301) comprises a microfluidic pump consisting of a linear actuator and support (3010) that houses a main body (3011) with an external inlet of compressed air and internal compressed air circulation channels; a polymer syringe (3017) with Polycaprolactone (PCL) arranged in said main body and pressed by said actuator of said microfluidic pump; a cap (3012) of said main body (3011) that prevents the passage of air, with a central opening through which the needle of said polymer syringe is arranged (3017), an outer cap (3014) to be coupled with the main body, a nozzle (3013) to be coupled with the outer cap (3014) and a central opening for the passage of the polymer syringe needle (3017) with a larger diameter annular separation for the passage of compressed air, where the inner face of the nozzle (3013) and the outer face of the cap (3012) form a pointed ring nozzle for an air outlet channel; where said plug (3012) and said outer cap (3014) form a compressed air circulation channel, of substantially constant cross-sectional area, from the internal compressed air circulation channels to the air outlet channel of said end annular nozzle, said circulation channel follows a mainly curved route.

3. The installation according to claim 1, **characterized in that** said electromechanical and robotic means of displacement of said stem positioner assembly comprises a horizontal movable stem positioner support (204); a stem positioner lifting pillar (205); a stem positioner lifting mobile support (206); stem positioner cross rails (207); a stem positioner transverse mobile support (208) and lower rails for the shank positioner (203).

4. The installation according to claim 1, **characterized in that** said electromechanical and robotic means of displacement of said nanofiber ejector positioner assembly comprise an ejector positioner lifting pillar (302); an ejector positioner angular variation unit (304); an ejector positioner lifting mobile support (303).

5. The installation according to claim 1, **characterized in that** it comprises an air compressor (305) located outside said enclosure (10) and which is connected to said nanofiber ejector assembly (301) by means of a line or hose for compressed air (306).

6. The installation according to claim 1, **characterized in that** a compressed air line available in the enclosure (10) can supply compressed air to said nanofiber ejector assembly (301) in place of said hose (306).

7. The installation according to claim 1, **characterized in that** said first, second and third immersion tubes (401, 402, 403) arranged are arranged in a support and collector of immersion tubes (404) to recover spills of polymeric solution and facilitate the maintenance of the apparatus.

8. The installation according to claim 1, **characterized in that** said immersion container (501) in said support structure (100), is supported by a container positioning assembly with electromechanical and robotic displacement means (504, 505) to place said immersion tube in a position of use (F) and in a position of withdrawal (G).

9. The installation according to claim 1, **characterized in that** said UV light curing means comprise a UV LED panel mounted directly on the structure (100) to illuminate said core stem of the vascular prosthesis (202) when it is located in said fourth position (D) and immersed in said immersion container (501).

10. The installation according to claim 1, **characterized in that** said UV light curing media (506, 507, 508) comprise a UV light power supply (506); a UV light guide (507) fed by said power supply (506) and supported on a UV light holder (508) in said structure (100).

11. The installation according to claim 1, **characterized in that** the stem positioner assembly can locate the stem (202) in the respective positions (A, B, C, D and E), thanks to the controlled displacement of the horizontal movable stem positioner support (204) to carry out immersion stages in different solutions of the respective containers (401, 402, 403 and 501).

12. The installation according to claim 1, **characterized in that**, when the stem positioner assembly is positioned in position (E), the nanofiber ejector positioner assembly can arrange the nanofiber ejector assembly (301) in the position (H) of the first height with a first angular orientation, thanks to said ejector positioner angular variation unit (304) and said mobile positioner lifting support of ejector (303) and in the position (I)9 of the second height with a second angular orientation thanks to the angular variation unit of the ejector positioner (304).

13. The installation according to claim 1, **characterized in that** said stem positioner assembly comprises a 6-axis robotic manipulator arm (210) as electromechanical and robotic displacement means, and said nanofiber ejector positioning assembly also includes a 6-axis robotic manipulator arm (310) as electromechanical and robotic displacement means, both being robotic manipulator arms (210, 310) controlled by said control console (600).

14. The installation according to claim 1, **characterized in that** said containers (401, 402, 403, 501) with solution for immersion are tubes arranged vertically, and the first, second, third and fourth position (A, B, C, D) of the stem (202) is vertically aligned and on a first, second, third and fourth respective vertical tubes (401, 402, 403, 501).

15. The installation according to claim 14, **characterized in that** said immersion container (501) comprises a lid with automatic closure (502) and a rotary actuator (503) that allows the immersion container (501) to rotate on a transverse axis, for the agitation of the solution in the container (501).

16. The installation according to claim 1, **characterized in that** the containers (401, 402, 403, 501) with solution for immersion are horizontal channels and the first, second, third and fourth position (A, B, C, and D) of the stem (202) is aligned longitudinally with these channels, where the stem positioning assembly is assembled with said set of mandrel or rotary support (201) arranged with its axis of rotation and orienting a core stem (2020) horizontal, parallel or slightly inclined with respect to the containers (401, 402, 403, 501), and where the horizontal movable stem positioner support (204) is located in such a way as to be able to position the stem (2020) in a fifth position (E); and where the ejector positioner assembly also includes a horizontal mobile ejector positioner support (306) on a set of horizontal rails (307) arranged parallel to the stem and containers (401, 402, 403, 501), so that the blown nanofiber ejector assembly (301) can travel the length of the stem (2020) and project at least one nanofiber filament onto said stem (2020), from a first position (H) and from a second position (I), where said first and second positions vary in height and angle of incidence of the filament on the stem (2020).

17. The installation according to claim 16, **characterised in that** said core stem (2020) may include a notch (2021) that allows the stem to be further lowered through a passage opening (4012) of the containers (401, 402, 403, 501).

18. The installation according to claim 16, **characterized in that** said stem positioner assembly comprises a 6-axis robotic manipulator arm (210) as electromechanical and robotic displacement means, and said nanofiber ejector positioner assembly also includes a 6-axis robotic manipulator arm (310) as electromechanical and robotic displacement means, both being robotic manipulator arms (210, 310) controlled by said control console (600).
